# EUROPEAN PATENT APPLICATION

(11) **EP 0 558 156 A2**
(43) Date of publication of application: **01.09.1993**
(21) Application number: 93201034.1
(22) Date of filing: 14.12.1990
(51) Int. Cl.: C07D 211/56

(54) **Intermediates for 3-aminopiperidine derivates**

(30) Priority: 04.01.1990 WO PCT/US90/00116
(62) Divisional of application: 90313680.2
(71) Applicant: PFIZER INC., New York, N.Y. 10017 (US)
(72) Inventor: Desai, Manoj C., Mystic, Connecticut (US); Rosen, Terry J., East Lyme, Connecticut (US)
(74) Representative: Moore, James William, Dr.

(57) **Abstract**

3-Amino-2-phenylpiperidine is a valuable intermediate for the preparation of certain 3-aminopiperidine substance P antagonists. (Divisional of application no 90313680.2).

## Description

This application is a divisional of European patent application no 90313680.2 filed 14th December 1990 and published as EP-A-0436334.

According to the specification of EP-A-0436334 we describe and claim certain aminopiperidine derivatives and related compounds as substance P antagonists. The present invention relates to the compounds 3-amino-2-phenylpiperidine and (2S,3S)-3-amino-2-phenylpiperidine which are valuable synthetic intermediates for the preparation of certain of the compounds claimed therein.

EP-A-0436334 claims compounds having the formula-: 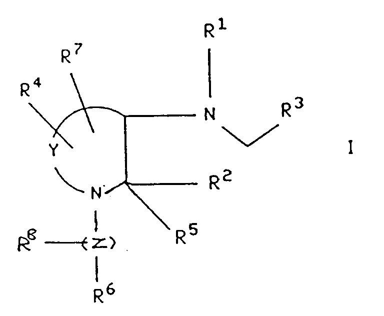
wherein Y is (CH₂)ₙ wherein n is an integer from 1 to 4, and wherein any one of the carbon-carbon single bonds in said (CH₂)ₙ may optionally be replaced by a carbon-carbon double bond, and wherein any one of the carbon atoms of said (CH₂)ₙ may optionally be substituted with R⁴, and wherein any one of the carbon atoms of said (CH₂)ₙ may optionally be substituted with R⁷;
Z is (CH₂)ₘ wherein m is an integer from 0 to 6, and wherein any one of the carbon-carbon single bonds of (CH₂)ₘ may optionally be replaced by a carbon-carbon double bond or a carbon-carbon triple bond, and any one of the carbon atoms of said (CH₂)ₘ may optionally be substituted with R⁸;
R¹ is hydrogen or (C₁-C₈) alkyl optionally substituted with hydroxy, (C₁-C₄)alkoxy or fluoro;
R² is a radical selected from hydrogen, (C₁-C₆) straight or branched alkyl, (C₃-C₇) cycloalkyl wherein one of the CH₂ groups in said cycloalkyl may optionally be replaced by NH, oxygen or sulfur; aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; phenyl-(C₂-C₆)- alkyl, benzhydryl and benzyl, wherein each of said aryl and heteroaryl groups and the phenyl moieties of said benzyl, phenyl-(C₂-C₆)-alkyl and benzhydryl may optionally be substituted with one or more substituents independently selected from halo, nitro, (C₁-C₆) alkyl, (C₁-C₆) alkoxy, trifluoromethyl, amino, (C₁-C₆)-alkylamino, 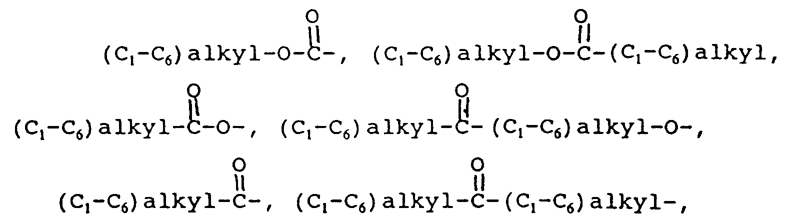
di-(C₁-C₆)alkylamino, 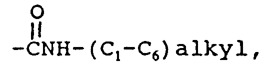 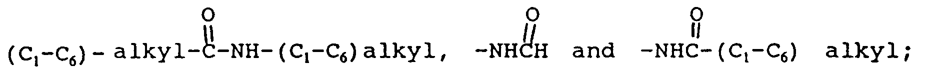
and wherein one of the phenyl moieties of said benzhydryl may optionally be replaced by naphthyl, thienyl, furyl or pyridyl;
R⁵ is hydrogen, phenyl or (C₁-C₆)alkyl;
or R² and R⁵, together with the carbon to which they are attached, form a saturated ring having from 3 to 7 carbon atoms wherein one of the CH₂ groups in said ring may optionally be replaced by oxygen, NH or sulfur;
R³ is aryl selected from phenyl and naphthyl; heteroaryl selected from indanyl, thienyl, furyl, pyridyl, thiazolyl, isothiazolyl, oxazolyl, isoxazolyl, triazolyl, tetrazolyl and quinolyl; and cycloalkyl having 3 to 7 carbon atoms wherein one of the (CH₂) groups in said cycloalkyl may optionally be replaced by NH, oxygen or sulfur; wherein each of said aryl and heteroaryl groups may optionally be substituted with one or more substituents, and said (C₃-C₇) cycloalkyl may optionally be substituted with one or two substituents, each of said substituents being independently selected from halo, nitro, (C₁-C₆) alkyl, (C₁-C₆) alkoxy, trifluoromethyl, amino, (C₁-C₆) alkylamino, 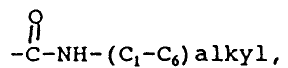 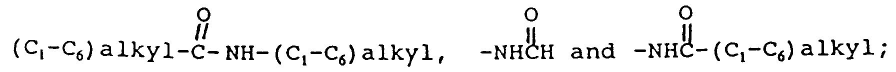
and
R⁴ and R⁷ are each independently selected from hydroxy, hydrogen halo, amino, oxo, cyano, methylene, hydroxymethyl, halomethyl, (C₁-C₆)alkylamino, di-(C₁-C₆)alkylamino, (C₁-C₆)alkoxy, 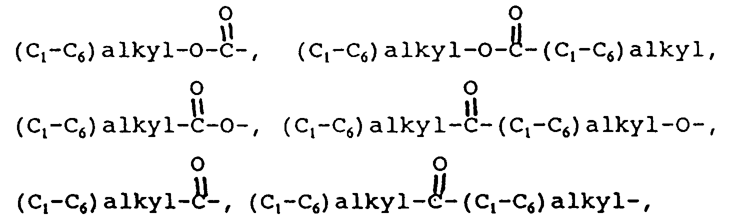
and the radicals set forth in the definition of R²,
R⁶ is 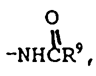
-NHCH₂R⁹, SO₂R⁹ or one of the radicals set forth in any of the definitions of R², R⁴ and R⁷;
R⁸ is oximino (=NOH) or one of the radicals set forth in any of the definitions of R², R⁴ and R⁷;
R⁹ is (C₁-C₆)alkyl, hydrogen, phenyl or phenyl (C₁-C₆)alkyl;
with the proviso that (a) when m is 0, R⁸ is absent, (b) when R⁴, R⁶, R⁷ or R⁸ is as defined in R², it cannot form, together with the carbon to which it is attached, a ring with R⁵, (c) when R⁴ and R⁷ are attached to the same carbon atom, then either each of R⁴ and R⁷ is independently selected from hydrogen, fluoro and (C₁-C₆) alkyl, or R⁴ and R⁷, together with the carbon to which they are attached, form a (C₃-C₆) saturated carbocyclic ring that forms a spiro compound with the nitrogen-containing ring to which they are attached, and (d) one of R² and R⁵ must be other than hydrogen or unsubstituted (C₁-C₆)alkyl.

According to EP-A-0436334 the compounds of formula I are prepared by a variety of routes including the route set out in Scheme I-: 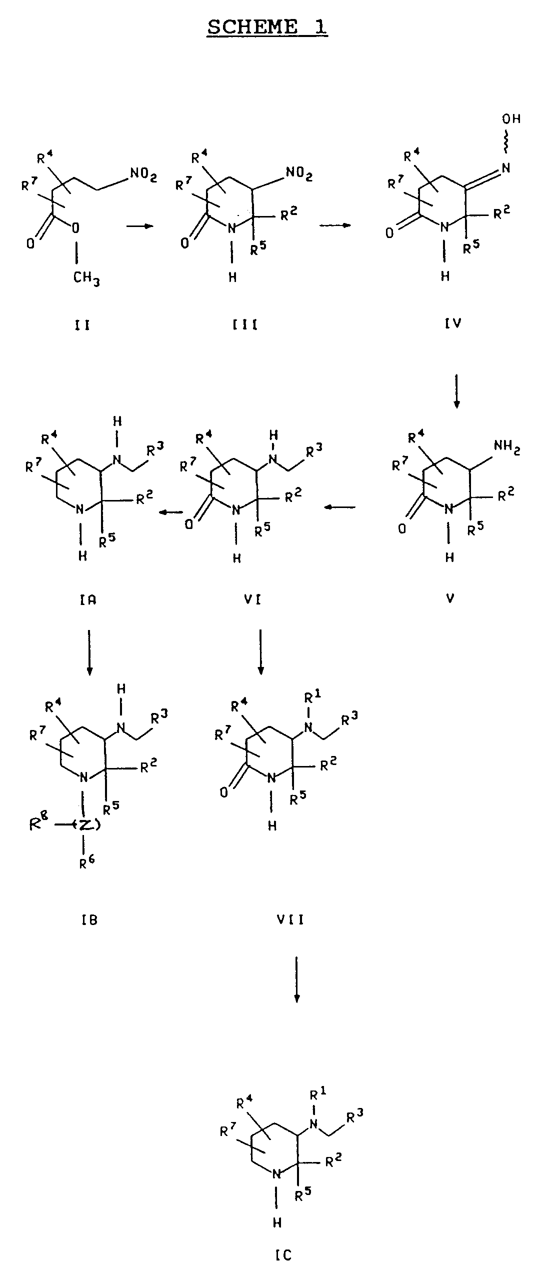
Compounds having the formula IA wherein R⁴, R⁵ and R⁷ are each hydrogen and R² is phenyl may be prepared, alternatively, by reductive amination of 3-amino-2-phenylpiperidine, using the appropriate aldehyde of the formula R³CHO, as described in EP-A-0436 334 for converting compounds of the formula V to the corresponding compounds of the formula VI.

According to the present invention there is provided the starting material for this reaction, 3-amino-2-phenylpiperidine. This compound may be prepared by hydrogenolysis of 3-(2-methoxybenzylamino)-2-phenylpiperidine. The hydrogenolysis reaction is usually carried out using a catalyst such as palladium on carbon or palladium hydroxide, in an inert solvent such as acetic acid or an alcoholic solvent, at a temperature from about 0°C to about 50°C. It is preferably carried out at about room temperature in a methanol/ethanol solvent. It is also preferable to conduct this reaction in the presence of a mineral acid such as hydrochloric or sulfuric acid.

The above two step process for preparing compounds of the formula IA wherein R⁴, R⁵ and R⁷ are each hydrogen and R² is phenyl from 3-(2-methoxybenzylamino)-2-phenylpiperidine preserves the stereochemistry at the "2" and "3" positions of the piperidine ring. It therefore may be used to produce either pure enantiomer or a racemic mixture of the product of formula IA from a sample of 3-(2-methoxybenzylamino)-2-phenylpiperidine having the same stereochemistry. Similarly, the first step of the above process may be used to produce either pure enantiomer or a racemic mixture of 3-amino-2-phenylpiperidine.

An alternative method of preparing racemic 3-amino-2-phenylpiperidine is by reducing 3-amino-2-phenylpyridine. This reduction is generally accomplished using either sodium in alcohol, lithium aluminum hydride/aluminum trichloride, electrolytic reduction or hydrogen in the presence of a metal catalyst. The reduction with sodium is generally conducted in a boiling alcohol, preferably butanol, at a temperature from about 20°C to about the reflux temperature of the solvent, preferably at about 120°C. The reduction with lithium aluminum hydride/aluminum trichloride is usually carried out in ether, THF or dimethoxyethane, preferably ether, at a temperature from about 25°C to about 100°C, preferably at about room temperature. The electrolytic reduction is conducted, preferably, at room temperature, but temperatures from about 10°C to about 60°C are also suitable.

Hydrogenation in the presence of a metal catalyst is the preferred method of reduction. Suitable hydrogenation catalysts include palladium, platinum, nickel and rhodium. The preferred catalyst for hydrogenation is platinum oxide. The reaction temperature may range from about 10°C to about 50°C, with about 25°C being preferred. The hydrogenation is generally carried out at a pressure from about 1.5 to about 4 atmospheres, preferably at about 3.0 atmospheres.

### EXAMPLE 1

### (+)-(2S,3S)-3-Amino-2-phenylpiperidine

In a bottle were placed 10 g of 10% palladium-carbon, 100 ml of methanol, 150 ml of ethanol, 3.5 ml of concentrated hydrochloric acid and 5 g of the hydrochloride salt of (+)S,S-cis-3-(2-methoxybenzylamino)-2-phenylpiperidine (see EP-A-0436334, Example 64). The mixture was shaken under hydrogen (40 p.s.i.) overnight, 5 g of additional catalyst was added to the system and the mixture was shaken under hydrogen for 3 days. The mixture was diluted with water, filtered through diatomaceous earth (Celite (trademark) and the Celite (trademark) was rinsed with H₂O. The filtrate was concentrated to remove most of the alcohol, the remaining liquid was extracted with chloroform and the chloroform extracts were dried (sodium sulfate) and concentrated to obtain 2.16 g of the title compound.
[α]_{D} (HCl salt) = + 62.8° (C=0.46, MeOH).
¹H NMR (CDCl₃) δ 1.68 (m, 4H), 2.72 (m, 1H), 2.95 (m, 1H), 3.16 (m, 1H), 3.80 (d, 1H, J=3), 7.24 (m, 5H).
HRMS Calc,d for C₁₁H₁₆N₂:176.1310. Found: 176.1309. Calc'd for C₁₁H₁₆N₂·2HCl·1/3H₂O: C, 51.78; H, 7.36; N, 10.98. Found: C, 51.46; H, 7.27; N, 10.77.

## Claims

1. 3-Amino-2-phenylpiperidine.

2. (2S,3S)-3-Amino-2-phenylpiperidine.

3. A process for preparing 3-amino-2-phenylpiperidine by hydrogenolysis of 3-(2-methoxybenzylamino)-2-phenylpiperidine.

## Claims (Claims for the following Contracting State(s): ES, GR)

1. A process for preparing 3-amino-2-phenylpiperidine by hydrogenolysis of 3-(2-methoxybenzylamino)-2-phenylpiperidine.
